# EUROPEAN PATENT SPECIFICATION

(11) **EP 4 410 246 B1**
(45) Date of publication and mention of the grant of the patent: **11.11.2026**
(21) Application number: 22880724.4
(22) Date of filing: 20.09.2022
(51) Int. Cl.: A61F 2/44, A61K 35/28, A61K 35/32, A61L 27/18, A61L 27/20, A61L 27/22, A61L 27/36, A61L 27/38, A61L 27/40, A61P 19/08, A61P 43/00, C12N 5/0775

(54) **BONE REPAIR DEVICE AND SURGICAL KIT**
KNOCHENREPARATURVORRICHTUNG UND CHIRURGISCHES KIT
DISPOSITIF DE RÉPARATION OSSEUSE ET TROUSSE CHIRURGICALE

(30) Priority: 27.09.2021 JP 2021156438
(43) Date of publication of application: 07.08.2024
(73) Proprietor: Fujita Academy, Toyoake-shi, Aichi 470-1192 (JP)
(72) Inventor: FUJITA Nobuyuki, Toyoake-shi, Aichi 470-1192 (JP); HORI Hideo, Toyoake-shi, Aichi 470-1192 (JP)
(74) Representative: Heyerhoff Geiger GmbH & Co. KG
(86) International application number: PCT/JP2022/034909
(87) International publication number: WO 2023/063028

(56) References cited:
- JP-A- 2012 192 105
- JP-A- 2019 022 651
- JP-A- 2020 195 330
- JP-A- 2021 516 074
- US-A1- 2009 105 825
- US-A1- 2013 338 790
- US-A1- 2019 201 212
- US-A1- 2020 093 612

## Description

### [Technical Field]

The present invention relates to a bone repair device and a surgical kit.

### [Background Art]

Posterior lumber interbody fusion has been widely used for treatment of spinal canal stenosis and spondylolisthesis. In the posterior lumber interbody fusion, an intervertebral disk is dissected, an implant called an intervertebral cage is inserted into a part where the intervertebral disk has been dissected, a screw and a rod are used to fuse adjacent vertebral bodies, and thereby stenosis of a spinal canal is eliminated. To ensure the stability between vertebral bodies after surgery, the adjacent vertebral bodies and the intervertebral cage need to be firmly agglutinated to each other, and improvement for this purpose has been applied to intervertebral cages. For example, Patent Literature 1 discloses an intervertebral cage in which a hole to accommodate bone fragments of a human body is formed.

### [Citation List]

### [Patent Literature]

Patent Literature 1: Japanese Patent Application Laid-Open No. 2012-19919

### [Summary of Invention]

### [Technical Problem]

In the intervertebral cage of Patent Literature 1, it requires a certain period of time for treatment before the intervertebral cage is agglutinated to the adjacent vertebral bodies by a bone repair effect caused by bone fragments. Examples of bone repair devices and materials are disclosed in US 2009/105825 A1, JP 2012 192105 A, JP 2020 195330 A and US 2013/338790 A1. Thus, to realize early rehabilitation into society of a patient, there is a demand for a further reduction in a treatment period. Further, such a challenge is present not only in a case of agglutination of adjacent vertebral bodies with an intervertebral cage but also in a case of repairment of bones in other sites.

The present invention has been made based on such a background, and an object is to provide a bone repair device according to independent claim 1, and a surgical kit according to claim 8, that promote bone repairment. Preferred embodiments of the invention are set forth in the dependent claims.

### [Solution to Problem]

To achieve the above object, a bone repair device according to the first aspect of the present invention includes:
an implant provided with a space inside into which a body fluid is allowed to flow from outside; and
a nonwoven fabric which is accommodated in the space of the implant and to which mesenchymal cells or osteoblasts differentiated from the mesenchymal cells are adhered.

The mesenchymal stem cells may be collected from a patient in which the implant is to be implanted.

The fiber diameter of the nonwoven fabric may be within a range from 0.1 µm to 30 µm.

The fiber density of the nonwoven fabric may be within a range from 0.2 g/m² to 150 g/m².

The nonwoven fabric may be formed of a material containing at least any one of polylactic acid, polyglycolic acid, silk fibroin, poly-ε-caprolactone, chitin, and chitosan.

The bone repair device may further include a bone fragment accommodated in the space of the implant together with the nonwoven fabric.

The implant may include
a main body, the space being formed inside the main body, and
a through-hole provided in the main body, penetrating from a surface of the main body to the space, and configured to allow a body fluid to flow into the space.

The implant may be an intervertebral cage to be inserted in an area between a pair of vertebral bodies, the area being resulted from dissection of an intervertebral disk.

To achieve the above object, a surgical kit according to the second aspect of the present invention includes:
an implant provided with a space inside into which a body fluid is allowed to flow from outside; and
a nonwoven fabric package including a nonwoven fabric, the nonwoven fabric being configured to be accommodated in the space of the implant, stem cells or osteoblasts differentiated from the stem cells being adhered to the nonwoven fabric, and the nonwoven fabric being packed.

### [Advantageous Effect]

According to the present invention, a bone repair device and a surgical kit that promote bone repairment can be provided.

### [Brief Description of Drawings]

[FIG. 1A] FIG. 1A is a plan view illustrating a configuration of a bone repair device according to an embodiment of the present invention.
[FIG. 1B] FIG. 1B is a front view illustrating the configuration of the bone repair device according to the embodiment of the present invention.
[FIG. 2A] FIG. 2A is a schematic diagram illustrating an anatomical configuration of a spine.
[FIG. 2B] FIG. 2B is a schematic diagram illustrating a view in which the bone repair device according to the embodiment of the present invention is embedded between a pair of vertebral bodies.
[FIG. 3] FIG. 3 is a diagram of an example of a nonwoven fabric according to the embodiment of the present invention, which is taken by a scanning electron microscope.
[FIG. 4] FIG. 4 is a diagram illustrating a procedure to adhere mesenchymal stem cells to the nonwoven fabric according to the embodiment of the present invention.

### [Description of Embodiments]

A bone repair device and a surgical kit according to an embodiment of the present invention will be described below in detail with reference to the drawings. In respective drawings, the same or similar features are labeled with the same reference symbol.

The bone repair device is an instrument that is embedded in a bone defect part, bears against the load from the bone defect part, and promotes bone repairment at the bone defect part. Although a case where the bone repair device is embedded in a defect part resulted from dissection of an intervertebral disk in posterior lumber interbody fusion will be described below as an example, the bone repair device may be formed in various shapes or sizes in accordance with the form of the bone defect part.

A bone defect part is a defect that has occurred in a bone or cartilage within a body and, for example, includes not only a site from which a bone or cartilage has been artificially removed but also a fracture site, a cartilage damaged site, or other defect sites caused by bone diseases. Further, bone repairment is intended to include not only restoration of a bone tissue or a function that was originally present in a bone defect part but also replacement of all of or a part of a bone defect part with an artificial material or recovery of a part of a function that was originally present in the bone defect part.

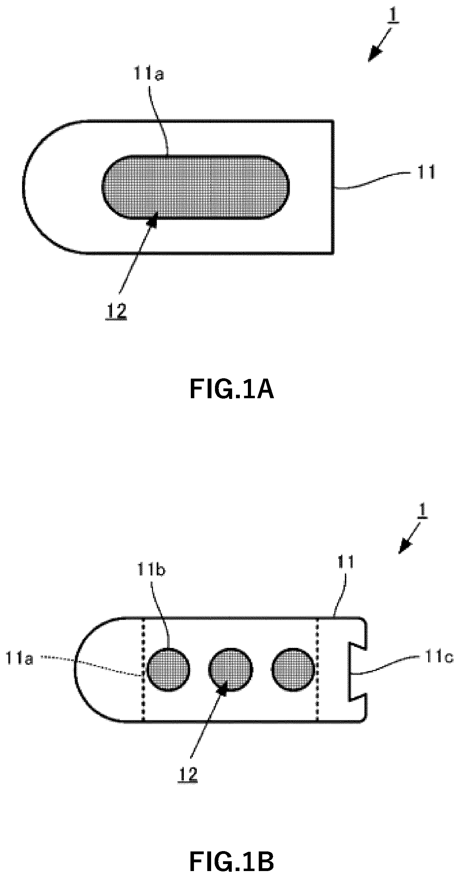

FIG. 1A and FIG. 1B are a plane view and a front view illustrating the configuration of a bone repair device 1 according to the embodiment of the present invention, respectively. The bone repair device 1 includes: an intervertebral cage 11 to be inserted in an area between adjacent vertebral bodies where an intervertebral disk has been resected by posterior lumber interbody fusion; and a nonwoven fabric 12 accommodated in the intervertebral cage 11 and having mesenchymal stem cells or osteoblasts differentiated from the mesenchymal stem cells adhered thereto.

The intervertebral cage 11 is an example of an implant embedded in a body and is a cage-shaped instrument that is mounted between adjacent two vertebral bodies of a vertebra and bears against the load from the vertebral bodies. The intervertebral cage 11 is formed of a biocompatible material, for example, a metal material such as titanium or a titanium alloy. For example, the intervertebral cage 11 is formed by applying machining to a block-shaped solid material and has a rigidity to the degree that is not deformed even when subjected to the load between the adjacent vertebral bodies. Further, for example, an anti-slip part formed of a groove or a protrusion may be formed in or to the top face and the bottom face of the intervertebral cage 11 so that the intervertebral cage 11 does not slip off between the vertebral bodies.

The intervertebral cage 11 is opened in the top face and the bottom face and includes an accommodation hole 11a in which a nonwoven fabric 12 is accommodated and a plurality of pores 11b each extending from the side face to the accommodation hole 11a. The accommodation hole 11a is an example of the space to accommodate the nonwoven fabric 12 and is formed in an oval shape or an elliptical shape in planar view, for example. The pore 11b is an example of a through-hole, and a plurality of pores are provided spaced apart from each other in the side face so that a body fluid containing cells or nutrients is evenly supplied to the nonwoven fabric 12 accommodated in the accommodation hole 11a. A plurality of pores 11b are also provided on the back side of FIG. 1B in the same manner as on the front side.

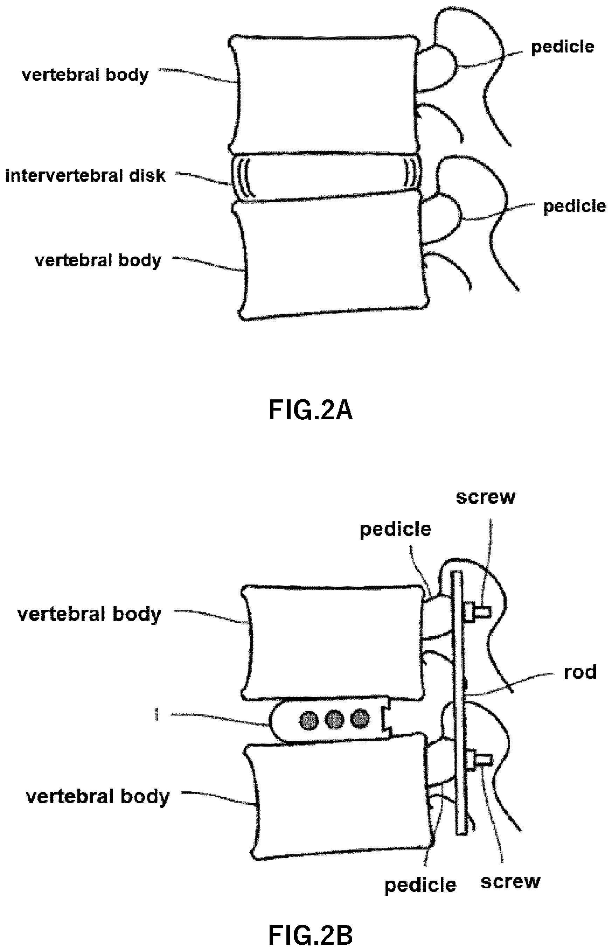

The tip of the intervertebral cage 11 has a round shape so that the intervertebral cage 11 is pushed into the gap between adjacent vertebral bodies. On the other hand, a mount groove 11c into which a long mount jig can be attached is formed to the base end of the intervertebral cage 11. When the operator operates the mount jig to push the intervertebral cage 11 into the gap between the adjacent vertebral bodies that is resulted from removal of the intervertebral disk illustrated in FIG. 2A, the intervertebral cage 11 of the bone repair device 1 is thereby embedded between the adjacent vertebral bodies, as illustrated in FIG. 2B.

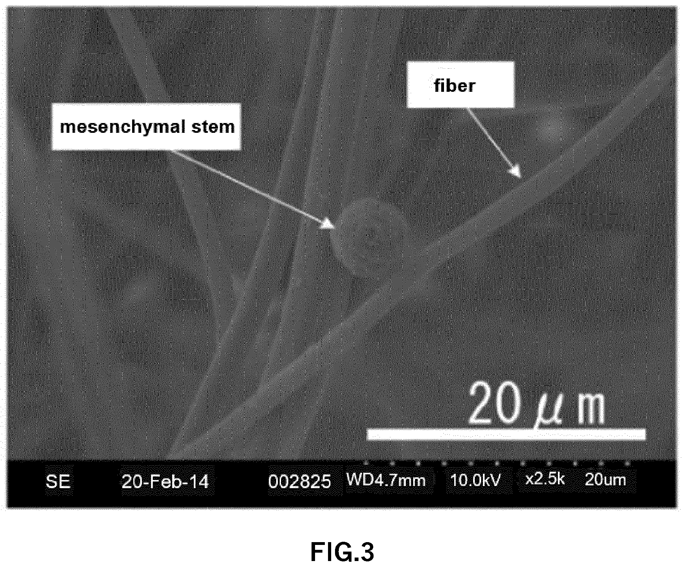

FIG. 3 is a diagram of an example of the nonwoven fabric 12 according to the embodiment, which is taken by a scanning electron microscope. The nonwoven fabric 12 is a sheet in which fine fibers are intertwined without being woven, and mesenchymal stem cells can be adhered to each of the fibers. The nonwoven fabric 12 can be deformed into any shape and thus is suitable to be accommodated inside the intervertebral cage 11.

In the nonwoven fabric 12, the fiber diameter and the fiber density have been adjusted so that mesenchymal stem cells are adhered to every fiber and a cell suspension or a body fluid flows into the space between the fibers. To cause cells to be adhered to fibers, it is preferable to reduce the fiber diameter of the nonwoven fabric 12. In contrast, an excessively small fiber diameter of the nonwoven fabric 12 may reduce the strength, and an excessively large fiber density may cause clogging of cells and make it difficult to handle the nonwoven fabric 12. In view of the adhesion of mesenchymal stem cells and easiness of manufacturing of the nonwoven fabric 12, the fiber diameter of the nonwoven fabric 12 is preferably within a range from 0.1 µm to 30 µm, and more preferably within a range from 0.1 µm to 3 µm. Further, the fiber density of the nonwoven fabric 12 is preferably within a range from 0.2 g/m² to 150 g/m².

The nonwoven fabric 12 is formed of a bioabsorbable material, for example, polylactic acid, polyglycolic acid, polyethylene glycol, silk fibroin, poly-ε-caprolactone, chitin, chitosan, copolymers of polylactic acid and polyglycolic acid, copolymer of polylactic acid and polyethylene glycol, or any combination of two or more thereof. In terms of promoting production of a growth factor in mesenchymal stem cells, the nonwoven fabric 12 is preferably formed of polylactic acid, silk fibroin, poly-ε-caprolactone, chitin, or chitosan.

Mesenchymal stem cells are somatic stem cells having the ability to differentiate into cells belonging to the mesenchymal. As the mesenchymal stem cells to be adhered to the nonwoven fabric 12, such a mesenchymal stem cell is selected that may promote growth of osteoblasts at the bone defect part by producing the growth factor of the osteoblasts or may differentiate into the osteoblasts. The mesenchymal stem cells to be adhered to the nonwoven fabric 12 may be any of those derived from fat, derived from periosteum, derived from synovial membrane, derived from cancellous bone, derived from bone marrow, derived from amniotic membrane, derived from umbilical cord blood, and derived from placenta as long as it satisfies the conditions described above. Further, multiple types of mesenchymal stem cells may be adhered together to the nonwoven fabric 12.

The mesenchymal stem cells to be adhered to the nonwoven fabric 12 may be collected from the patient and then cultured in vitro. To culture mesenchymal stem cells in vitro, for example, a medium containing Fetal Bovine Serum (FBS) or human serum, preferably, human serum derived from the patient or a serum-free medium can be used. The mesenchymal stem cells cultured in a medium may be adhered directly to the nonwoven fabric 12 or may be adhered to the nonwoven fabric 12 after the mesenchymal stem cells are differentiated to express osteoblasts. To express osteoblasts, for example, a differentiation-inducing factor can be added to the medium of mesenchymal stem cells.

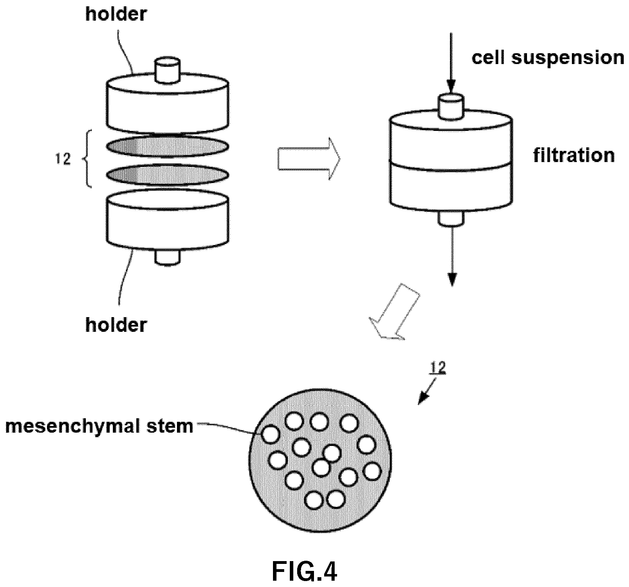

To adhere mesenchymal stem cells to the nonwoven fabric 12, it is preferable to cause a cell suspension containing the mesenchymal stem cells to be in contact with the nonwoven fabric for a while. A specific procedure to adhere mesenchymal stem cells to the nonwoven fabric will be described below with reference to FIG. 4.

First, the nonwoven fabric 12 is mounted between a pair of holders forming a filtration device. Each holder is configured so that a cell suspension can flow out of the holders or flow into the holders. Because the nonwoven fabric 12 is interposed between the pair of holders, the cell suspension comes into contact with the nonwoven fabric 12 when the cell suspension is caused to flow from one holder to the other holder. Note that multiple sheets of the nonwoven fabric 12 may be interposed between the pair of holders.

Next, the cell suspension containing mesenchymal stem cells is caused to slowly drop from above to filtrate the cell suspension through the nonwoven fabric 12. In response, the mesenchymal stem cells are gradually adhered to the fibers of the nonwoven fabric 12. Although the exact mechanism is unclear, after the cell suspension containing mesenchymal stem cells is filtrated through the nonwoven fabric 12 over time, the mesenchymal stem cells adhered to the nonwoven fabric 12 are activated.

Note that, if the mesenchymal stem cells have been adhered to the nonwoven fabric 12, production of the growth factor having a bone tissue repair effect from the mesenchymal stem cells can be expected, and it is therefore not necessarily required to use the procedure illustrated in FIG. 4 to activate mesenchymal stem cells.

A manufacturing method of the bone repair device 1 according to the embodiment will be described below. First, the nonwoven fabric 12 with mesenchymal stem cells adhered thereto is acquired. To acquire the nonwoven fabric 12 with mesenchymal stem cells adhered thereto, for example, it is preferable to culture mesenchymal stem cells collected from a human body of a patient or a person other than the patient and adhere the cultured mesenchymal stem cells to the nonwoven fabric 12 in advance. It is preferable to cut the nonwoven fabric 12 into a size suitable for the intervertebral cage 11 at any timing before or after adhering the mesenchymal stem cells.

The nonwoven fabric 12 with the mesenchymal stem cells adhered thereto may be directly accommodated inside the intervertebral cage 11 or may be stored as a nonwoven fabric package packed with a wrapping material. The nonwoven fabric package including the nonwoven fabric 12 packed as a package with the stem cells being adhered thereto may be provided to the operator as a surgical kit together with the intervertebral cage 11.

Next, the nonwoven fabric 12 with the mesenchymal stem cells adhered thereto is accommodated in the intervertebral cage 11. For example, it is preferable to pinch the nonwoven fabric 12 by tweezers and push the nonwoven fabric 12 into the intervertebral cage 11 so as not to cause unbalanced positioning of the nonwoven fabric 12 in the intervertebral cage 11. Note that the intervertebral cage 11 accommodating the nonwoven fabric 12 may be directly embedded between the adjacent vertebral bodies, or the intervertebral cage 11 accommodating the nonwoven fabric 12 may be packed and then stored until surgery.

The above is the flow of the manufacturing method of the bone repair device 1.

A procedure of posterior lumber interbody fusion performed by the operator using the bone repair device 1 according to the embodiment will be described below. First, the operator makes an incision on a skin of a target site and develops both sides of the spine posterior tissue from the incised part, and inserts screws into a plurality of pedicles located aligned vertically as illustrated in FIG. 2A, respectively. Next, decompression to relieve pressure to spine nerves is performed. Next, the intervertebral disk is dissected from one side or both sides, and one or two bone repair devices 1 are embedded as replacement, as illustrated in FIG. 2B. Next, a vertically arranged rod is fixed to the plurality of screws inserted in the plurality of pedicles to fix the spine from the back side. Next, the skin is sutured to close the incised part.

The above is a series of procedures of the posterior lumber interbody fusion.

Since the nonwoven fabric 12 is accommodated in the accommodation hole 11a of the intervertebral cage 11 in the bone repair device 1 embedded between the adjacent vertebral bodies by posterior lumber interbody fusion, proliferation or growth of osteoblasts is promoted from the nonwoven fabric 12 as a scaffold due to the effect of the mesenchymal stem cells or the osteoblasts adhered to the nonwoven fabric 12. As a result, compared to an intervertebral cage not accommodating the nonwoven fabric 12, the adjacent vertebral bodies can be fixed earlier to each other, and earlier repairment of the spine can be realized.

As described above, the bone repair device 1 according to the embodiment includes the intervertebral cage 11 provided with a space inside into which a body fluid can flow from outside and the nonwoven fabric 12 which is accommodated in the space of the intervertebral cage 11 and to which mesenchymal stem cells or osteoblasts differentiated from the mesenchymal stem cells are adhered. Thus, early repairment of a bone defect part can be realized.

The present invention is not limited to the embodiment described above, and modifications stated below are possible.

### [Modified Example]

Although the intervertebral cage 11 has the accommodation hole 11a and the pores 11b in the embodiment described above, the present invention is not limited thereto. The intervertebral cage 11 may be of any shape as long as the space into which a body fluid can flow from outside is provided therein.

Although the intervertebral cage 11 is formed of a metal material in the embodiment described above, the present invention is not limited thereto. For example, a resin material such as Poly Ether Ether Ketone (PEEK) may be employed, and fiber-reinforced plastics, ceramics, or artificial bones may be employed.

Although mesenchymal stem cells are adhered to the nonwoven fabric 12 in order to promote bone repairment in the embodiment described above, the present invention is not limited thereto. The stem cells to be adhered to the nonwoven fabric 12 may be, for example, Embryonic Stem (ES) cells or induced Pluripotent Stem (iPS) cells.

Although the nonwoven fabric 12 with mesenchymal stem cells adhered thereto is directly accommodated in the intervertebral cage 11 in the embodiment described above, the present invention is not limited thereto. For example, the nonwoven fabric 12 with mesenchymal stem cells adhered thereto may also be used with a gelling agent added thereto. As the gelling agent, for example, collagen, gelatin, an extracellular matrix mixture, fibrin, polysaccharides, or chondroitin can be used. As the extracellular matrix mixture, for example, Matrigel (registered trademark) by Corning Inc. can be used, and as the polysaccharides, for example, agarose can be used.

Although the nonwoven fabric 12 with mesenchymal stem cells adhered thereto is accommodated inside the intervertebral cage 11 in the embodiment described above, the present invention is not limited thereto. For example, bone fragments collected from a human body in addition to the nonwoven fabric 12 may be accommodated together. The bone fragments are obtained by crushing a bone collected from a human body and are preferably obtained by crushing a bone (a local bone or an iliac bone) collected from the subject patient. The shape or the size of the bone fragment is set taking the shape or the size of the intervertebral cage 11 or the embedding portion of the intervertebral cage 11 into consideration. To prevent bone fragments from scattering within the body, the bone fragments may be wrapped by the nonwoven fabric 12 and accommodated inside the intervertebral cage 11. Further, at a point of time before or after the nonwoven fabric 12 is accommodated inside the intervertebral cage 11, an agent to promote bone repairment or inhibit an immune response may be held in the nonwoven fabric 12.

Although the case where the intervertebral cage 11 is embedded in a defect site between adjacent vertebral bodies has been described as an example in the above embodiment, the present invention is not limited thereto. The implant is not limited to the intervertebral cage 11, and any implant applicable to other sites may be employed as long as a space into which a body fluid can flow from outside is provided therein. For example, to join bones to each other in osteotomy of long bones, an implant accommodating therein the nonwoven fabric with mesenchymal stem cells adhered thereto may be embedded between the bones.

The embodiment described above is exemplary illustration, the present invention is not limited thereto, and various embodiments are possible within the scope not departing from the spirit of the invention recited in the claims. The components described in the embodiment or the modified examples can be combined in any manner. Further, inventions equivalent to the invention recited in the claims are also included in the present invention.

The present application is based on Japanese Patent Application No. 2021-156438 filed on September 27, 2021 and includes the specification, the claims, the drawings, and the abstract thereof. The entirety of the disclosure in the Japanese Patent Application listed above is included in the present specification by reference.

### [Industrial Applicability]

The bone repair device and the surgical kit of the present invention are useful for promoting bone repairment.

### [List of Reference Symbols]

- 1: bone repair device
- 11: intervertebral cage
- 11a: accommodation hole
- 11b: pore
- 11c: mount groove
- 12: nonwoven fabric

## Claims

1. A bone repair device (1) comprising:
an implant provided with a space inside into which a body fluid is allowed to flow from outside; and
a nonwoven fabric (12) which is accommodated in the space of the implant and which comprises intertwined fibers, wherein mesenchymal stem cells are adhered to intertwined fibers,
wherein a shape of the nonwoven fabric (12), when the mesenchymal stem cells are adhered, is different from a shape of the space of the implant, and
wherein the nonwoven fabric (12) can be deformed into any shape, and thus the nonwoven fabric (12) to which the mesenchymal stem cells are adhered is suitable to be accommodated in the space.

2. The bone repair device (1) according to claim 1, wherein the fiber diameter of the nonwoven fabric (12) is within a range from 0.1 µm to 30 µm.

3. The bone repair device (1) according to claim 1 or 2, wherein the fiber density of the nonwoven fabric (12) is within a range from 0.2 g/m² to 150 g/m².

4. The bone repair device (1) according to any one of claims 1 to 3, wherein the nonwoven fabric (12) is formed of a material containing at least any one of polylactic acid, polyglycolic acid, silk fibroin, poly-ε-caprolactone, chitin, and chitosan.

5. The bone repair device (1) according to any one of claims 1 to 4 further comprising a bone fragment accommodated in the space of the implant together with the nonwoven fabric (12).

6. The bone repair device (1) according to any one of claims 1 to 5, wherein the implant comprises
a main body having the space formed inside, and
a through-hole provided in the main body, penetrating from a surface of the main body to the space, and configured to allow a body fluid to flow into the space.

7. The bone repair device (1) according to any one of claims 1 to 6, wherein the implant is an intervertebral cage (11) to be inserted in an area between a pair of vertebral bodies, the area being resulted from dissection of an intervertebral disk.

8. A surgical kit used for the bone repair device (1) according to any one of claims 1 to 7, wherein the surgical kit comprising:
an implant according to any one of claims 1 to 7; and
a nonwoven fabric (12) package in which the nonwoven fabric (12) according to any one of claims 1 to 7 being packed.

## Patentansprüche

1. Knochenreparaturvorrichtung (1), die Folgendes umfasst:
ein Implantat, in dem ein Raum bereitgestellt ist, in den von außen ein Körperfluid strömen kann; und
einen Vliesstoff (12), der im Raum des Implantats aufgenommen ist und der verflochtene Fasern umfasst, wobei an den verflochtenen Fasern mesenchymale Stammzellen haften,
wobei sich eine Form des Vliesstoffs (12), wenn die mesenchymalen Stammzellen anhaften, von einer Form des Raums des Implantats unterscheidet, und
wobei der Vliesstoff (12) in irgendeine Form verformt werden kann, und folglich der Vliesstoff (12), an dem die mesenchymalen Stammzellen anhaften, geeignet ist, in dem Raum aufgenommen zu werden.

2. Knochenreparaturvorrichtung (1) nach Anspruch 1, wobei der Faserdurchmesser des Vliesstoffs (12) innerhalb eines Bereichs von 0,1 µm bis 30 µm liegt.

3. Knochenreparaturvorrichtung (1) nach Anspruch 1 oder 2, wobei die Faserdichte des Vliesstoffs (12) innerhalb eines Bereichs von 0,2 g/m² bis 150 g/m² liegt.

4. Knochenreparaturvorrichtung (1) nach einem der Ansprüche 1 bis 3, wobei der Vliesstoff (12) aus einem Material gebildet ist, das wenigstens eines von Polymilchsäure, Polyglykolsäure, Seidenfibroin, Poly-ε-Caprolacton, Chitin und Chitosan enthält.

5. Knochenreparaturvorrichtung (1) nach einem der Ansprüche 1 bis 4, die ferner ein Knochenfragment umfasst, das zusammen mit dem Vliesstoff (12) in dem Raum des Implantats aufgenommen ist.

6. Knochenreparaturvorrichtung (1) nach einem der Ansprüche 1 bis 5, wobei das Implantat Folgendes umfasst:
einen Hauptkörper, der den darin ausgebildeten Raum aufweist, und
ein Durchgangsloch, das in dem Hauptkörper bereitgestellt ist, das von einer Oberfläche des Hauptkörpers in den Raum eindringt und das konfiguriert ist, einem Körperfluid zu ermöglichen, in den Raum zu fließen.

7. Knochenreparaturvorrichtung (1) nach einem der Ansprüche 1 bis 6, wobei das Implantat ein Zwischenwirbelkäfig (11) ist, der in einen Bereich zwischen einem Paar von Wirbelkörpern eingesetzt werden soll, wobei der Bereich aus einer Dissektion einer Zwischenwirbelscheibe resultiert.

8. Chirurgisches Kit, das für die Knochenreparaturvorrichtung (1) nach einem der Ansprüche 1 bis 7 verwendet wird, wobei das chirurgische Kit Folgendes umfasst:
ein Implantat nach einem der Ansprüche 1 bis 7; und
eine Verpackung des Vliesstoffs (12), in der der Vliesstoff (12) nach einem der Ansprüche 1 bis 7 verpackt ist.

## Revendications

1. Dispositif de réparation osseuse (1) comprenant :
un implant pourvu d'un espace à l'intérieur duquel un liquide corporel peut s'écouler depuis l'extérieur ; et un non-tissé (12) qui est logé dans l'espace de l'implant et qui comprend des fibres entrelacées, dans lequel des cellules souches mésenchymateuses sont amenées à adhérer aux fibres entrelacées,
dans lequel une forme du non-tissé (12), lorsque les cellules souches mésenchymateuses ont adhéré, est différente d'une forme de l'espace de l'implant et
dans lequel le non-tissé (12) peut être déformé en une forme quelconque et donc le non-tissé (12) auquel les cellules souches mésenchymateuses ont adhéré convient pour être logé dans l'espace.

2. Dispositif de réparation osseuse (1) selon la revendication 1, dans lequel le diamètre des fibres du non-tissé (12) se situe dans une plage allant de 0,1 µm à 30 µm.

3. Dispositif de réparation osseuse (1) selon la revendication 1 ou 2, dans lequel la densité des fibres du non-tissé (12) se situe dans une plage allant de 0,2 g/m² à 150 g/m².

4. Dispositif de réparation osseuse (1) selon l'une quelconque des revendications 1 à 3, dans lequel le non-tissé (12) est constitué d'un matériau en contenant au moins un quelconque parmi le poly(acide lactique), le poly(acide glycolique), la fibroïne de soie, la poly-ε-caprolactone, la chitine et le chitosane.

5. Dispositif de réparation osseuse (1) selon l'une quelconque des revendications 1 à 4 comprenant en outre un fragment osseux logé dans l'espace de l'implant conjointement avec le non-tissé (12).

6. Dispositif de réparation osseuse (1) selon l'une quelconque des revendications 1 à 5, dans lequel l'implant comprend
un corps principal à l'intérieur duquel est formé l'espace et
un trou traversant ménagé dans le corps principal, pénétrant depuis une surface du corps principal vers l'espace et conçu pour permettre à un liquide corporel de s'écouler dans l'espace.

7. Dispositif de réparation osseuse (1) selon l'une quelconque des revendications 1 à 6, dans lequel l'implant est une cage intervertébrale (11) destinée à être insérée dans une zone entre une paire de corps vertébraux, la zone résultant de la dissection d'un disque intervertébral.

8. Trousse chirurgicale utilisée pour le dispositif de réparation osseuse (1) selon l'une quelconque des revendications 1 à 7, la trousse chirurgicale comprenant :
un implant selon l'une quelconque des revendications 1 à 7 ; et
un emballage de non-tissé (12) dans lequel le non-tissé (12) selon l'une quelconque des revendications 1 à 7 est emballé.
